(19) Europäisches Patentamt
European Patent Office
Office européen des brevets

(11) Veröffentlichungsnummer : **0 096 801**
**B1**

(12) # EUROPÄISCHE PATENTSCHRIFT

(45) Veröffentlichungstag der Patentschrift : **09.09.87**

(51) Int. Cl.⁴ : **C 07 D249/08**, C 07 D233/60// A01N43/50, A01N43/64

(21) Anmeldenummer : **83105405.1**

(22) Anmeldetag : **01.06.83**

(54) **Azolylmethyl-ketone, Verfahren zu ihrer Herstellung und ihre Verwendung als Zwischenprodukte.**

(30) Priorität : **12.06.82 DE 3222220**

(43) Veröffentlichungstag der Anmeldung : **28.12.83 Patentblatt 83/52**

(45) Bekanntmachung des Hinweises auf die Patenterteilung : **09.09.87 Patentblatt 87/37**

(84) Benannte Vertragsstaaten : **BE CH DE FR GB IT LI**

(56) Entgegenhaltungen :
**DE-A- 2 737 489**
**DE-A- 2 833 194**
**DE-A- 2 929 602**
**DE-A- 2 937 595**
**DE-A- 3 028 330**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Jäger, Gerhard, Dr.**
**Gellertstrasse 18**
**D-5090 Leverkusen 1 (DE)**
Erfinder : **Jautelat, Manfred, Dr.**
**Müllersbaum 28**
**D-5093 Burscheid (DE)**
Erfinder : **Krämer, Wolfgang, Dr.**
**Am Eckbusch 39/45**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Azolylmethylketone, ein Verfahren zu deren Herstellung und ihre Verwendung als Zwischenprodukte zur Synthese von Hydroxyalkinyl-Derivaten, welche fungizide Eigenschaften besitzen.

Es ist bereits bekannt geworden, daß bestimmte Hydroxyalkyl-triazole, wie beispielsweise 2-(4-Biphenylyl)-1-(2,4-dichlorphenyl)-3-(1,2,4-triazol-1-yl)-2-propanol oder 2-(4-Biphenylyl)-1-(4-chlorphenyl)-3-(1,2,4-triazol-1-yl)-2-propanol, fungizide Eigenschaften aufweisen (vergleiche DE-A 29 20 374). Die Wirksamkeit dieser Verbindungen ist jedoch, insbesondere bei niedrigen Aufwandmengen und -konzentrationen, nicht immer ganz befriedigend.

Außerdem sind bereits zahlreiche Azolylmethylketone bekannt, in denen der mit der Carbonyl-Gruppe verbundene Rest für Alkyl, Halogenalkyl oder gegebenenfalls substituiertes Cycloalkyl steht (vgl. DE-A 27 37 489 ; DE-A 29 37 595, DE-A 29 29 602 und DE-A 30 28 330). Entsprechende Verbindungen, die einen Neopentyl- oder Cyanethyl-Rest, bzw. einen über $—C(CH_3)_2—$ gebundenen, gegebenenfalls substituierten Phenoxyethyl-Rest oder einen zweifach substituierten Cycloalkyl-Rest enthalten, werden jedoch nicht erwähnt.

In der DE-A 28 33 194 werden Azolylmethylketone offenbart, in denen als zusätzlicher Substituent an der Keto-Gruppe ein gegebenenfalls substituierter Phenyl-Rest vorhanden ist. Es werden aber keine Stoffe aufgeführt, die andere Reste als gegebenenfalls substituiertes Phenyl aufweisen.

Schließlich werden in der EP-A 0 094 572 Azolylketone beschrieben, in denen eine Phenoxymethyl-Gruppe über eine $—C(CH_3)_2$-Einheit mit der Carbonyl-Gruppe verknüpft ist. Entsprechende Verbindungen mit einer Phenoxyethyl-Gruppe an der betreffenden Stelle werden jedoch nicht aufgeführt.

Es wurden neue Azolylmethyl-ketone der Formel I

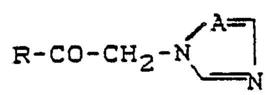

(I)

in welcher

A für ein Stickstoffatom oder die CH-Gruppe steht,

R für Adamantyl oder die Gruppierungen

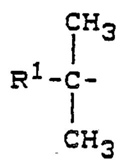

und

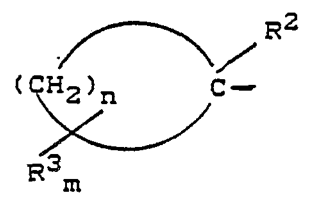

steht, wobei

$R^1$ für Neopentyl, Cyanethyl oder gegebenenfalls einfach bis dreifach, gleich oder verschieden substituiertes Phenoxyethyl steht, wobei als Phenylsubstituenten genannt seien : Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4 Kohlenstoffatomen in jedem Alkylteil, Nitro, Cyano, sowie Alkoxycarbonyl mit 1 bis 4 Kohlenstoffatomen im Alkoxyteil und gegebenenfalls durch Halogen substituiertes Phenyl oder Phenoxy ;

$R^2$ für geradkettiges oder verzweigtes Alkyl mit 1 bis 6 Kohlenstoffatomen steht,

$R^3$ für geradkettiges oder verzweigtes Alkyl mit 1 bis 4 Kohlenstoffatomen, Chlor oder Brom steht,

m für die Zahlen 1 oder 2 steht und

n für die Zahlen 3, 4, 5, 6 und 7 steht,

gefunden.

Weiterhin wurde gefunden, daß man die neuen Azolylmethylketone der Formel I enthält, indem man Halogenmethylketone der Formel II

$$R—CO—CH_2—Hal \qquad (II)$$

in welcher

R die oben angegebene Bedeutung hat und

Hal für Chlor oder Brom steht,

mit Azolen der Formel III

Siehe Formel, Seite 3

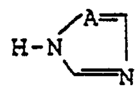

(III)

in welcher A die oben angegebene Bedeutung hat, in Gegenwart eines Verdünnungsmittels und in Gegenwart eines Säurebindemittels umsetzt.

Die neuen Azolylmethyl-ketone sind interessante Zwischenprodukte zur Herstellung von Pflanzenschutz-Wirkstoffen. So eignen sich die Stoffe der Formel I als Ausgangsstoffe zur Synthese von Hydroxyalkinyl-azolyl-Derivaten, welche sehr gute fungizide Wirksamkeit besitzen.

Überraschenderweise sind die Hydroxyalkinyl-azolyl-Derivate, die sich aus den erfindungsgemäßen Azolylmethylketonen der Formel I z. B. durch Umsetzung mit Propargylhalogeniden in Gegenwart von aktiviertem Aluminium herstellen lassen, den aus dem Stand der Technik bekannten Verbindungen 2-(4-Biphenylyl)-1-(2,4-dichlorphenyl)-3-(1,2,4-triazol-1-yl)-2-propanol und 2-(4-Biphenylyl)-1-(4-chlorphenyl)-3-(1,2,4-triazol-1-yl)-2-propanol bezüglich ihrer fungiziden Wirksamkeit überlegen.

Die erfindungsgemäßen Stoffe sind durch die Formel I allgemein definiert.

Bevorzugt sind diejenigen Verbindungen der Formel I, in denen R für Adamantyl oder die Gruppierungen

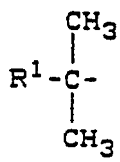

und

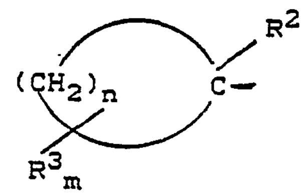

steht,

$R^1$ für Neopentyl, Cyanethyl oder gegebenenfalls einfach bis zweifach, gleich oder verschieden substituiertes Phenoxyethyl steht, wobei als Phenylsubstituenten genannt seien : Fluor, Chlor, Brom, Methyl, Ethyl, Isopropyl, tert.-Butyl, Methoxy, Ethoxy, Methylthio, Trifluormethyl, Trifluormethoxy, Trifluormethylthio, Nitro, Dimethylamino, Methoxycarbonyl sowie gegebenenfalls durch Chlor substituiertes Phenyl oder Phenoxy ;

$R^2$ für Methyl oder Ethyl steht ;

$R^3$ für Methyl, Ethyl, Isopropyl, tert.-Butyl oder Chlor steht ;

m für die Zahlen 1 oder 2 steht ; und

A und n für die in der Erfindungsdefinition angegebenen Bedeutungen stehen.

Im einzelnen seien außer den bei den Herstellungsbeispielen genannten Verbindungen folgende Verbindungen der Formel I genannt (A steht sowohl für ein Stickstoffatom als auch für die CH-Gruppe) :

(Siehe Tabelle Seite 4 f.)

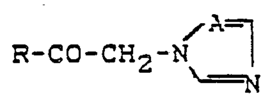 (I)

| R | R |
|---|---|
| 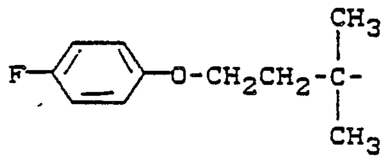 | 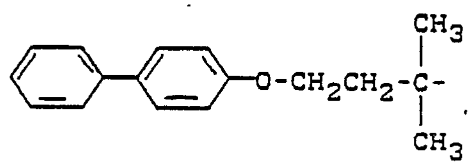 |
| 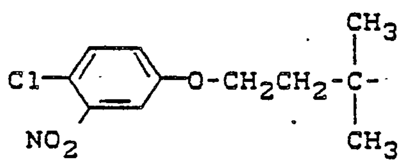 | 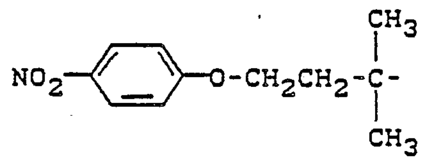 |
| 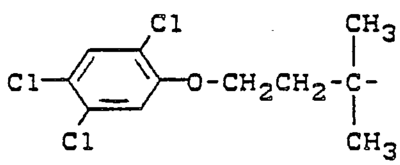 | 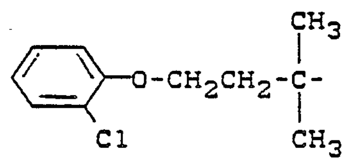 |
| 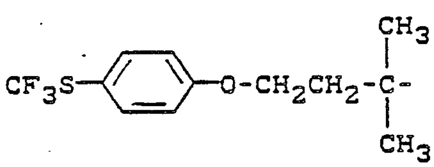 | 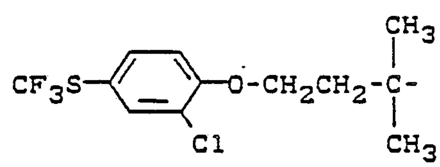 |
| 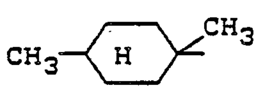 | 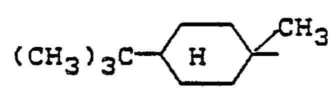 |
| 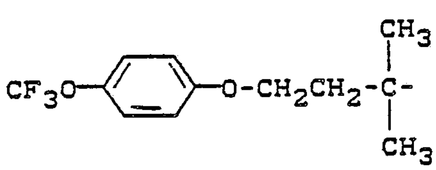 | 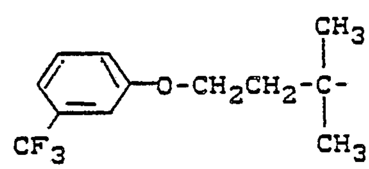 |

Verwendet man beispielsweise 1-Chlor-5-(2,4-dichlorphenoxy)-3,3-dimethyl-pentan-2-on und 1,2,4-Triazol als Ausgangsstoffe, so kann der Reaktionsablauf des erfindungsgemäßen Verfahrens durch das folgende Formelschema wiedergegeben werden :

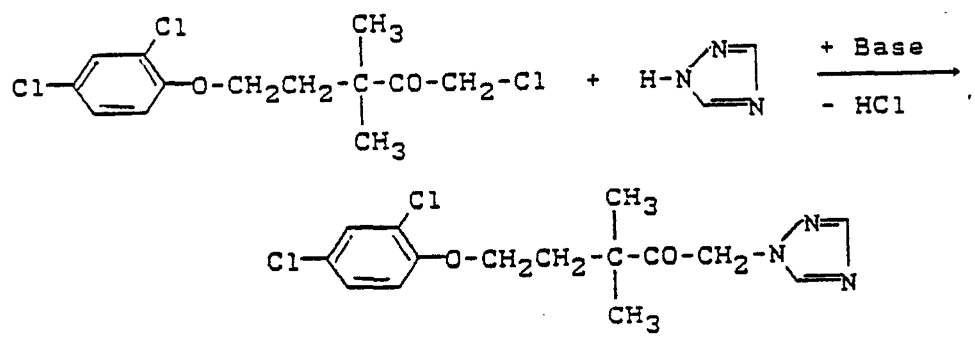


Die für die Durchführung des erfindungsgemäßen Verfahrens als Ausgangsstoffe benötigten Halogenmethyl-ketone sind durch die Formel II allgemein definiert. In dieser Formel steht R für diejenigen Bedeutungen, die bereits im Zusammenhang mit der Beschreibung der erfindungsgemäßen Stoffe der Formel I für diesen Substituenten genannt wurden.

Die Halogenmethyl-ketone der Formel II sind zum Teil bekannt (DE-A 30 49 461). Sie lassen sich nach bekannten Verfahren in einfacher Weise herstellen, indem man entsprechende Methylketone in üblicher Weise mit Chlor oder Brom in Gegenwart eines inerten organischen Lösungsmittels, wie beispielsweise Ether, chlorierten oder nichtchlorierten Kohlenwasserstoffen, bei Raumtemperatur umsetzt, oder mit üblichen Chlorierungsmitteln, wie beispielsweise Sulfurylchlorid, bei 20 bis 60 °C umsetzt.

Die Halogenmethylketone der Formel II können auch erhalten werden, indem man 1,1-Dichloralkene der Formel IV

$$R'—CH=CCl_2 \qquad (IV)$$

in welcher R' für die Bedeutung von R sowie die Gruppierung $Cl—CH_2CH_2—C(CH_3)_2—$ steht, mit Phenolen der Formel V

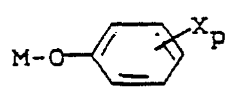
(V)

in welcher

M für ein Äquivalent eines Alkali- oder Erdalkalimetallions, insbesondere eines Natrium- oder Kaliumions steht,

X für Halogen, Alkyl oder Alkoxy mit jeweils 1 bis 3 Kohlenstoffatomen oder Phenyl steht und

p für die Zahlen 0, 1 oder 2 steht,

in Gegenwart eines inerten organischen Lösungsmittels, wie z. B. Dimethylformamid, bei Temperaturen zwischen 100 und 220 °C, gegebenenfalls unter erhöhtem Druck, umsetzt und die dabei erhaltenen Phenylether der Formel VI

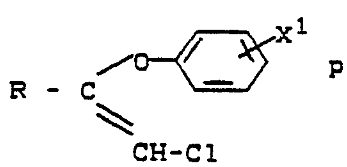
(VI)

in welcher R, $X^1$ und p die oben angegebene Bedeutung haben, in üblicher Weise mit Mineralsäuren, wie z. B. Schwefelsäure oder Salzsäure, und/oder mit organischen Säuren, wie z. B. Ameisensäure, bei 40 bis 100 °C hydrolysiert.

Die Herstellung von 1,1-Dichloralkenen der Formel IV ist bekannt. Sie erfolgt durch Addition von Alkylhalogeniden an Vinylidenchlorid in Gegenwart von sauren Katalysatoren [vgl. hierzu J. Am. Chem. Soc. 74, 2885 (1962)] unter gleichzeitiger Abspaltung von Halogenwasserstoff.

Für das erfindungsgemäße Verfahren kommen als Verdünnungsmittel inerte organische Lösungsmittel in Frage. Hierzu gehören vorzugsweise Ketone, wie Aceton, Methylethylketon und Methylbutylketon ; Alkohole, wie Ethanol, Isopropanol und Butanol ; aromatische Kohlenwasserstoffe, wie Benzol und Toluol ; Formamide und Sulfoxide, wie Dimethylformamid und Dimethylsulfoxid.

Das erfindungsgemäße Verfahren wird in Gegenwart eines Säurebindemittels vorgenommen. Man kann alle üblicherweise verwendbaren anorganischen oder organischen Säurebinder zugegeben, wie

Alkalicarbonate, beispielsweise Natriumcarbonat, Kaliumcarbonat und Natriumhydrogencarbonat, wie Alkali- und Erdalkalihydroxide, beispielsweise Kaliumhydroxid und Calciumhydroxid, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan, sowie auch einen entsprechendend Überschuß an Azol.

Die Reaktionstemperaturen können bei dem erfindungsgemäßen Verfahren in einem größeren Bereich variiert werden. Im allgemeinen arbeitet man bei Temperaturen zwischen 0 und 120 °C, vorzugsweise zwischen 20 und 90 °C. Zweckmäßigerweise arbeitet man beim Siedepunkt des jeweiligen Lösungsmittels.

Bei der Durchführung des erfindungsgemäßen Verfahrens setzt man auf 1 Mol der Verbindung der Formel II vorzugsweise 1 bis 4 Mol Azol und 1 bis 4 Mol Säurebinder ein. Zur Isolierung der Verbindungen der Formel I wird das Lösungsmittel abdestilliert und der Rückstand in üblicher Art und Weise aufgearbeitet.

Die erfindungsgemäßen Azolylmethyl-ketone der Formel I eignen sich als Zwischenprodukte zur Synthese von Hydroxyalkinyl-azolyl-Derivaten, welche fungizide Wirksamkeit besitzen.

Solche Hydroxyalkinyl-azolyl-Derivate der Formel VII

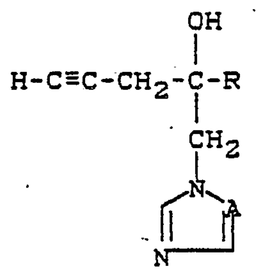

(VII)

in welcher A und R die oben angegebene Bedeutung haben, lassen sich herstellen, indem man beispielsweise Azolylmethyl-ketone der Formel I

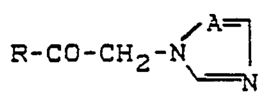

(I)

in welcher A und R die oben angegebene Bedeutung haben, mit Propargylhalogeniden der Formel VIII

$$HC{\equiv}C{-}CH_2{-}Hal' \qquad (VIII)$$

in welcher Hal' für Chlor oder Brom steht, in Gegenwart von aktiviertem Aluminium und in Gegenwart eines Verdünnungsmittels umsetzt.

Für die Umsetzung der erfindungsgemäßen Azolylmethylketone der Formel I mit Propargylhalogeniden der Formel VIII kommen als Verdünnungsmittel organische, aprotische Lösungsmittel in Frage, wie beispielsweise Diethylether oder Tetrahydrofuran.

Das oben angegebene Verfahren zur Herstellung von Hydroxyalkinyl-azolyl-Derivaten VII wird in Gegenwart von aktiviertem Aluminium durchgeführt. Diese Aktivierung erfolgt durch Zugabe katalytischer Mengen Quecksilber(II)- chlorid und Iod.

Die Reaktionstemperaturen können bei dem oben angegebenen Verfahren zur Herstellung von Hydroxyalkinyl-azolyl-Derivaten in einem größeren Bereich variiert werden. Im allgemeinen arbeitet man bei Temperaturen zwischen — 80 und + 100 °C, vorzugsweise zwischen — 70 und + 60 °C.

Bei der Durchführung des oben angegebenen Verfahrens zur Herstellung von Hydroxyalkinyl-azolyl-Derivaten VII setzt man auf 1 Mol Azolylmethyl-keton der Formel I 1 bis 2 Mol Propargylhalogenid der Formel VIII und 1 bis 1,5 Mol Aluminium sowie katalytische Mengen Quecksilber-(II)-chlorid und Iod ein. Die Isolierung der Verbindungen der Formel VII erfolgt in üblicher Weise.

Die aus den erfindungsgemäßen Stoffen herstellbaren Hydroxyalkinyl-azolyl-Derivate der Formel VII besitzen sehr gute fungizide Eigenschaften.

## Herstellungsbeispiele

### Beispiel 1

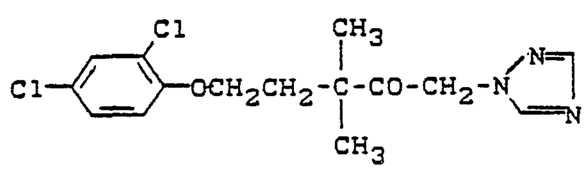

Zu einem Gemisch aus 13,8 g (0,1 Mol) Kaliumcarbonat und 13,8 g (0,2 Mol) 1,2,4-Triazol in 200 ml siedendem Aceton weden 30,9 g (0,1 Mol) 1-Chlor-5-(2,4-dichlorphenoxy)-3,3-dimethyl-pentan-2-on, gelöst in 80 ml Aceton, unter Rühren zugetropft. Man hält das Gemisch noch 3 Stunden am Sieden, kühlt auf 0 bis 10 °C ab, filtriert vom Salz ab und engt das Filtrat im Vakuum ein. Nach Anreiben des öligen Rückstandes mit etwas Petrolether erhält man 32,4 g (94,6 % der Theorie) 5-(2,4-Dichlorphenoxy)-3,3-dimethyl-1-(1,2,4-triazol-1-yl)-pentan-2-on als farblose Kristalle vom Schmelzpunkt 62 °C.

Herstellung des Ausgangsproduktes

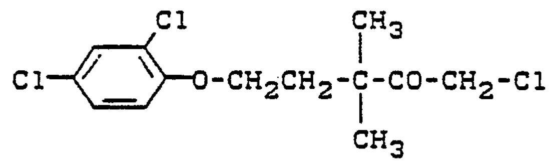

454 g (1 Mol) 1-Chlor-3,3-dimethyl-2,5-di-(2,4-dichlorphenoxy)-1-penten werden in 500 ml Ameisensäure und 50 ml konzentrierter Salzsäure 9 Stunden auf 100 °C erhitzt. Man verdünnt mit Methylenchlorid, schüttelt einmal mit Wasser und dreimal mit verdünnter Natronlauge aus. Nach dem Trocknen der Lösung wird das Lösungsmittel im Vakuum abgezogen. Man erhält 238 g (77 % der Theorie) rohes 5-(2,4-Dichlorphenoxy)-3,3-dimethyl-pentan-2-on, das langsam kristallisiert. Nach Waschen mit Petrolether hat das Produkt einen Schmelzpunkt von 55-58 °C.

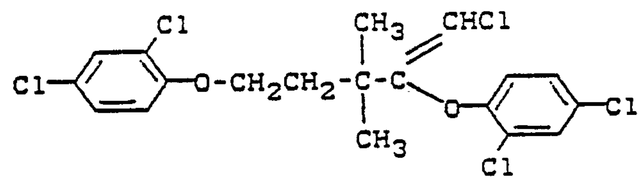

489 g (3 Mol) 2,4-Dichlorphenol werden in 1,2 l N-Methylpyrrolidon gelöst und mit 600 ml (3 Mol) einer 30 %igen Natriummethylatlösung versetzt. Bei 20 mbar destilliert man Methanol und 200 ml N-Methylpyrrolidon ab. 201 g (1 Mol) 1,1,5-Trichlor-3,3-Dimethyl-1-penten werden bei 200 °C und Atmosphärendruck langsam zugetropft. Man rührt 6 Stunden bei 200 °C nach. Die erhaltene Lösung wird mit Methylenchlorid verdünnt und mit verdünnter Natronlauge mehrfach ausgeschüttelt. Die getrocknete Lösung wird eingeengt und bei 0,1 mbar und 150 °C das restliche Lösungsmittel abgezogen. Man erhält 410 g (90 % der Theorie) 1-Chlor-3,3-dimethyl-1,5-di-(2,4-dichlorphenoxy)-penten als Öl.

NMR ($CDCl_3$) : $\delta$ = 1,2 (s, 6H), 2,1 (t, 2H, J = 7 Hz), 4,1 (t, 2H, J = 7 Hz), 5,95 (s, 1H), 6,75-7,4 (m, 6H).

## Beispiel 2

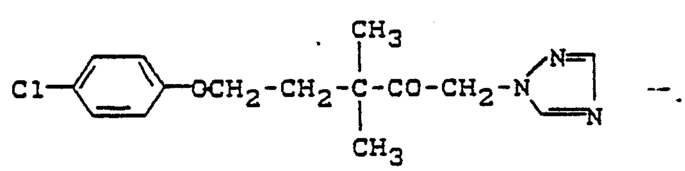

Zu einem Gemisch aus 27,6 g (0,2 Mol) Kaliumcarbonat und 27,6 g (0,4 Mol) 1,2,4-Triazol in 400 ml siedendem Aceton wird eine Lösung von 55 g (0,2 Mol) 1-Chlor-5-(4-chlorphenoxy)-3,3-dimethyl-pentan-2-on in 50 ml Aceton zugetropft. Man hält das Gemisch noch 3 Stunden am Sieden, kühlt auf 20 °C ab, filtriert und engt das Filtrat im Vakuum ein. Der ölige Rückstand wird in 250 ml Essigester aufgenommen und dreimal mit je 100 ml Wasser gewaschen. Nach dem Trocknen der organischen Phase über wasserfreiem Natriumsulfat wird im Vakuum eingedampft. Das verbleibende Rohprodukt (61 g) wird in 250 ml Aceton gelöst und mit einer Lösung von 57,6 g Naphthalin-1,5-disulfonsäure in 250 ml Aceton versetzt. Das auskristallisierte Salz (45,4 g ; Schmelzpunkt 188-190 °C) wird in einem Gemisch aus je 150 ml Dichlormethan und Wasser suspendiert und durch Zugabe von 10 %iger Natriumcarbonat-Lösung alkalisch gestellt. Man trennt die organische Phase ab, extrahiert die wäßrige Phase noch einmal mit 100 ml Dichlormethan und engt die vereinigten organischen Phasen im Vakuum ein. Man erhält 30,4 g (49,4 % der Theorie) 5-(4-Chlorphenoxy)-3,3-dimethyl-1-(1,2,4-triazol-1-yl)-pentan-2-on als schwach gelbliche Flüssigkeit vom Brechungsindex : $n_D^{20}$ = 1,545 7.

Herstellung des Ausgangsproduktes

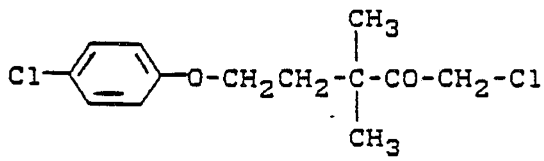

580 g (1,5 Mol) 1-Chlor-3,3-dimethyl-1,5-di-(4-chlor-phenoxy)-1-penten werden in 1 000 ml Ethanol und 300 ml konzentrierter Salzsäure 4 Stunden unter Rückfluß erhitzt. Man zieht das Ethanol im Vakuum ab, verdünnt mit Methylenchlorid und schüttelt mit Wasser, dann dreimal mit verdünnter Natronlauge aus. Die getrocknete Lösung wird im Vakuum vom Lösungsmittel befreit. Man erhält 363 g (88 % der Theorie) 1-Chlor-5-(4-chlorphenoxy)-3,3-dimethyl-pentan-2-on als Öl.

NMR ($CDCl_3$) : $\delta$ = 1,25 (s, 6H), 2,1 (t, 2H, J = 6 Hz), 3,9 (t, 2H, J = 6 Hz), 4,45 (s, 2H), 6,7-7,3 (m, 4H)

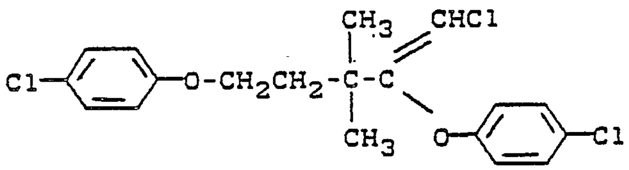

771 g (6 Mol) Chlorphenol werden in 2,4 l Dimethylformamid gelöst und mit 1,2 l (6 Mol) 30 %iger Natriummethylatlösung versetzt. Man zieht das Methanol im Vakuum ab und destilliert bei 20 mbar noch ca. 400 ml Dimethylformamid ab. Dann werden bei 150 °C langsam 403 g (2 Mol) 1,1,5-Trichlor-3,3-dimethyl-1-penten zugetropft und 8 Stunden unter Rückfluß erhitzt. Die Aufarbeitung erfolgt mit Methylenchlorid und Wasser sowie verdünnter Natronlauge. Nach Abziehen des Solvens im Vakuum wird bei 0,1 mbar und 150 °C andestilliert. Man erhält 646 g (84 % der Theorie) 1-Chlor-3,3-dimethyl-2,5-di-(4-chlorphenoxy)-1-penten als Öl.

NMR ($CDCl_3$) : $\delta$ = 1,2 (s, 6H), 2,0 (t, 2H, J = 7 Hz), 4,0 (t, 2H, 7 Hz), 5,9 (s, 1H), 6,75-7,35 ppm (m, 8H).

In analoger Weise und entsprechend dem erfindungsgemäßen Verfahren werden die nachfolgend aufgeführten Verbindungen der Formel I

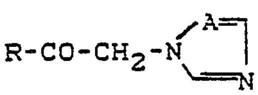

(I)

erhalten :

(Siehe Tabelle Seite 9 ff.)

| Bsp. Nr. | R | A | Schmelzpunkt (°C) bzw. Brechnungs-index $n_D^{20}$ |
|---|---|---|---|
| 3 | 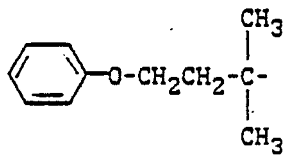 | N | Harz |
| 4 | 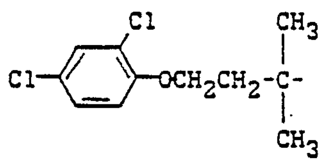 | CH | 46-48 |
| 5 | Adamantyl | N | 129 |
| 6 | 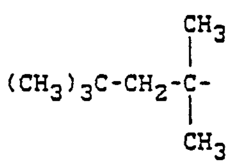 | N | 1,4851 |
| 7 | 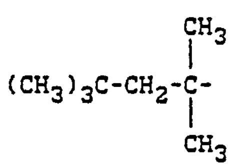 | CH | 54-56 |
| 8 | 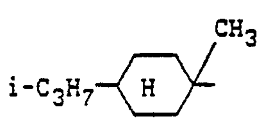 | N | 99-101 |
| 9 | 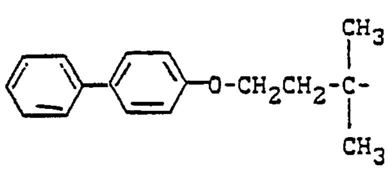 | CH | 132-34 |
| 10 | 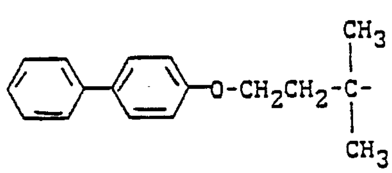 | N | 125-27 |

(Fortsetzung)

| Bsp. Nr. | R | A | Schmelzpunkt (°C) bzw. Brechnungsindex $n_D^{20}$ |
|---|---|---|---|
| 11 | 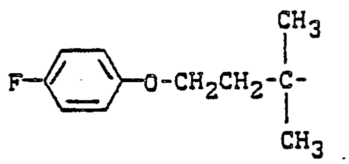 | CH | 1,5290 |
| 12 | 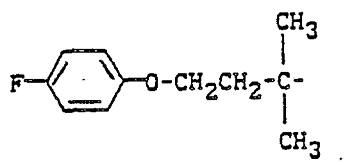 | N | 1,5238 |
| 13 | 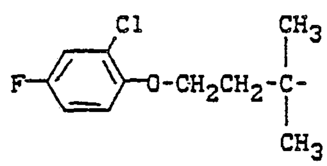 | CH | 69-70 |
| 14 | 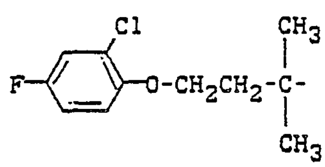 | N | 54-56 |
| 15 | 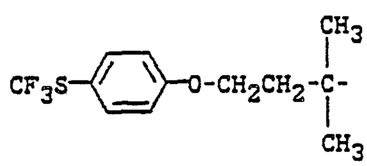 | CH | 1,5233 |
| 16 | 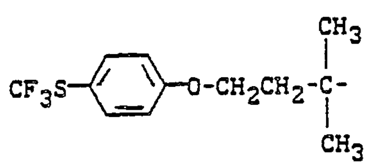 | N | 72-77 |
| 17 | 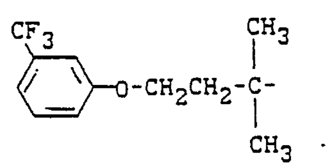 | CH | 1,5025 |

(Fortsetzung)

| Bsp. Nr. | R | A | Schmelzpunkt (°C) bzw. Brechnungs-index $n_D^{20}$ |
|---|---|---|---|
| 18 | 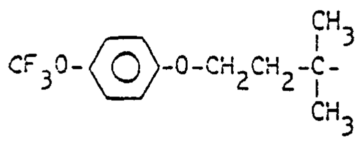 | CH | 1,4939 |
| 19 | 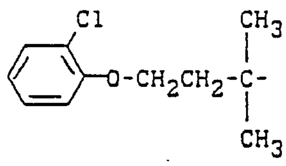 | CH | 78-79 |
| 20 | 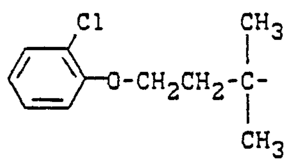 | N | 1,5441 |
| 21 | 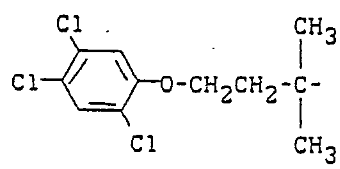 | CH | 127-32 |
| 22 | 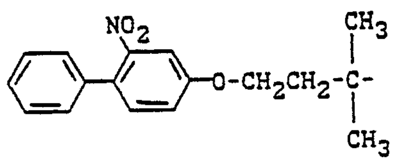 | CH | 82-83 |
| 23 | 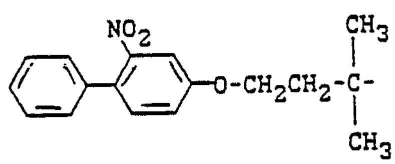 | N | 101-02 |
| 24 | 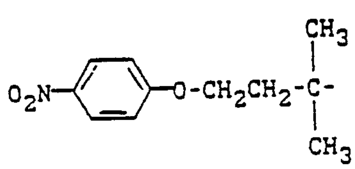 | N | 151-53 |
| 25 | 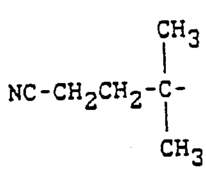 | N | 155-58(x HCl) |

Herstellung eines Folgeproduktes

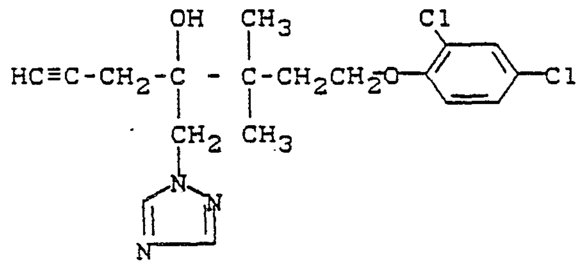

1,58 g (0,058 Mol) Aluminium (Schuppenform) werden mit 7,3 ml Tetrahydrofuran Überschichtet und mit einer katalytischen Menge (0,05 g) Quecksilber(II)-chlorid und einem Iodkristall versetzt. Nach 12-stündigem Stehen bei 20 °C werden bei 60 °C 10,3 g (0,087 Mol) Propargylbromid in 11 ml Tetrahydrofuran zugetropft. Man kühlt anschließend auf — 60 °C ab und tropft eine Lösung von 17,1 g (0,05 Mol) 5-(2,4-Dichlorphenoxy)-3,3-dimethyl-1-(1,2,4-triazol-1-yl)-pentan-2-on in 20 ml Tetrahydrofuran zu. Man läßt auf 0 °C erwärmen, hält noch eine Stunde bei dieser Temperatur und setzt 22 ml einer gesättigten, wäßrigen Ammoniumchlorid-Lösung zu. Anschließend wird filtriert, das Filtrat im Vakuum eingeengt und der Rückstand in 200 ml Essigester aufgenommen. Nach dreimaligem Waschen mit je 100 ml Wasser wird die organische Phase über Natriumsulfat getrocknet und anschließend im Vakuum eingeengt. Lösungsmittelreste werden bei 50 °C und 0,01 mbar entfernt. Man erhält 14,3 g (74,8 % der Theorie) 7-(2,4-Dichlorphenoxy)-5,5-dimethyl-4-(1,2,4-triazol-1-yl-methyl)-1-yl-methyl)-1-heptin-4-ol als bräunliches Öl vom Brechnungsindex $n_D^{20} = 1{,}564\,2$.

**Patentansprüche**

1. Azolylmethyl-ketone der allgemeinen Formel I

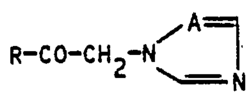 (I)

in welcher

A für ein Stickstoffatom oder die CH-Gruppe steht,

R für Adamantyl oder die Gruppierungen

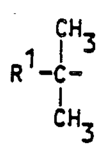 und 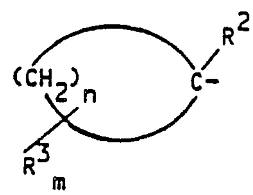 ,

steht, wobei

$R^1$ für Neopentyl, Cyanethyl oder gegebenenfalls einfach bis dreifach, gleich oder verschieden substituiertes Phenoxyethyl steht, wobei als Phenylsubstituenten genannt seien : Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4 Kohlenstoffatomen in jedem Alkylteil, Nitro, Cyano, sowie Alkoxycarbonyl mit 1 bis 4 Kohlenstoffatomen im Alkoxyteil und gegebenenfalls durch Halogen substituiertes Phenyl oder Phenoxy ;

$R^2$ für geradkettiges oder verzweigtes Alkyl mit 1 bis 6 Kohlenstoffatomen steht,

$R^3$ für geradkettiges oder verzweigtes Alkyl mit 1 bis 4 Kohlenstoffatomen, Chlor oder Brom steht,

m für die Zahlen 1 oder 2 steht und

n für die Zahlen 3, 4, 5, 6 und 7 steht.

2. Verbindungen der allgemeinen Formel I in Anspruch 1, wobei R für Adamantyl oder die Gruppierungen

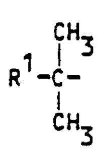 und 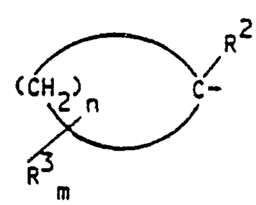 ,

steht ;

$R^1$ für Neopentyl, Cyanethyl oder gegebenenfalls einfach bis zweifach, gleich oder verschieden substituiertes Phenoxyethyl steht, wobei als Phenylsubstituenten genannt seien : Fluor, Chlor, Brom, Methyl, Ethyl, Isopropyl, tert.-Butyl, Methoxy, Ethoxy, Methylthio, Trifluormethyl, Trifluormethoxy, Trifluormethylthio, Nitro, Dimethylamino, Methoxycarbonyl sowie gegebenenfalls durch Chlor substituiertes Phenyl oder Phenoxy ;

$R^2$ für Methyl oder Ethyl steht ;

$R^3$ für Methyl, Ethyl, Isopropyl, tert.-Butyl oder Chlor steht ;

m für die Zahlen 1 oder 2 steht ; und

A für ein Stickstoffatom oder die CH-Gruppe steht und

n für die Zahlen 3, 4, 5, 6 und 7 steht.

3. Verfahren zur Herstellung von Azolylmethyl-ketonen der allgemeinen Formel I

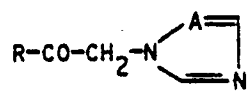

(I)

in welcher

A für ein Stickstoffatom oder die CH-Gruppe steht,

R für Adamantyl oder die Gruppierungen

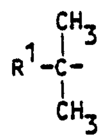

und

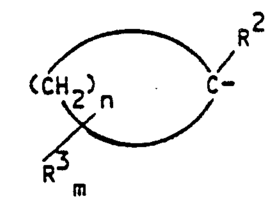

,

steht, wobei

$R^1$ für Neopentyl, Cyanethyl oder gegebenenfalls einfach bis dreifach, gleich oder verschieden substituiertes Phenoxyethyl steht, wobei als Phenylsubstituenten genannt seien : Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4 Kohlenstoffatomen in jedem Alkylteil, Nitro, Cyano, sowie Alkoxycarbonyl mit 1 bis 4 Kohlenstoffatomen im Alkoxyteil und gegebenenfalls durch Halogen substituiertes Phenyl oder Phenoxy ;

$R^2$ für geradkettiges oder verzweigtes Alkyl mit 1 bis 6 Kohlenstoffatomen steht,

$R^3$ für geradkettiges oder verzweigtes Alkyl mit 1 bis 4 Kohlenstoffatomen, Chlor oder Brom steht,

m für die Zahlen 1 oder 2 steht und

n für die Zahlen 3, 4, 5, 6 und 7 steht,

dadurch gekennzeichnet, daß man Halogenmethylketone der Formel II

$$R—CO—CH_2—Hal \qquad (II)$$

in welcher

R die oben angegebene Bedeutung hat und

Hal für Chlor oder Brom steht,

mit Azolen der Formel III

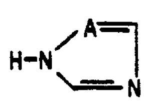

(III)

in welcher A die oben angegebene Bedeutung hat, in Gegenwart eines Verdünnungsmittels und in Gegenwart eines Säurebindemittels umsetzt.

4. Verwendung von Azolylmethyl-ketonen der allgemeinen Formel I in Ansprüchen 1 und 2 als Zwischenprodukte zur Herstellung von Hydroxyalkinyl-azolyl-Derivaten mit fungizider Wirksamkeit.

**Claims**

1. Azolylmethyl ketones of the general formula I

See formula, page 14

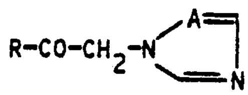 (I)

in which

A represents a nitrogen atom or the CH group,

R represents adamantyl or the groupings

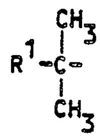 and 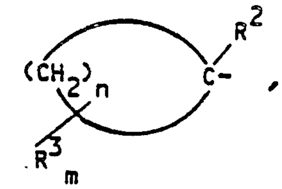 ,

wherein

$R^1$ represents neopentyl, cyanoethyl or phenoxyethyl which is optionally mono- to trisubstituted by identical or different substituents, the phenyl substituents which may be mentioned being : halogen, alkyl with 1 to 4 carbon atoms, alkoxy and alkylthio with in each case 1 to 2 carbon atoms, halogenoalkyl, halogenoalkoxy and halogenoalkylthio with in each case 1 to 2 carbon and 1 to 5 identical or different halogen atoms, cyclohexyl, dialkylamino with 1 to 4 carbon atoms in each alkyl part, nitro, cyano, and alkoxycarbonyl with 1 to 4 carbon atoms in the alkoxy part and optionally halogen-substituted phenyl or phenoxy ;

$R^2$ represents straight-chain or branched alkyl with 1 to 6 carbon atoms,

$R^3$ represents straight-chain or branched alkyl with 1 to 4 carbon atoms, chlorine or bromine,

m represents the numbers 1 or 2 and

n represents the numbers 3, 4, 5, 6 and 7.

2. Compounds of the general formula I in Claim 1, wherein R represents adamantyl or the groupings

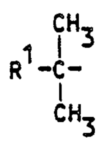 and 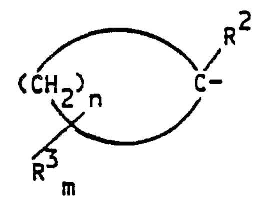 ,

$R^1$ represents neopentyl, cyanoethyl or phenoxyethyl which is optionally mono- to disubstituted by identical or different substituents, the phenyl susbtituents which may be mentioned being : fluorine, chlorine, bromine, methyl, ethyl, isopropyl, tert.-butyl, methoxy, ethoxy, methylthio, trifluoromethyl, trifluoromethoxy, trifluoromethylthio, nitro, dimethylamino, methoxycarbonyl and optionally chlorine-substituted phenyl or phenoxy ;

$R^2$ represents methyl or ethyl ;

$R^3$ represents methyl, ethyl, isopropyl, tert.-butyl or chlorine ;

m represents the numbers 1 or 2 ; and

A represents a nitrogen atom or the CH group and

n represents the numbers 3, 4, 5, 6 and 7.

3. Process for the preparation of azolylmethyl ketones of the general formula I

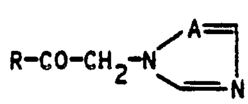 (I)

in which

A represents a nitrogen atom or the CH group,

R represents adamantyl or the groupings

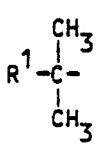 and 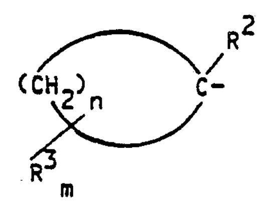 ,

wherein

$R^1$ represents neopentyl, cyanoethyl or phenoxyethyl which is optionally mono- to trisubstituted by identical or different substituents, the phenyl substituents which may be mentioned being : halogen, alkyl with 1 to 4 carbon atoms, alkoxy and alkylthio with in each case 1 to 2 carbon atoms, halogenoalkyl, halogenoalkoxy and halogenoalkylthio with in each case 1 to 2 carbon and 1 to 5 identical or different halogen atoms, cyclohexyl, dialkylamino with 1 to 4 carbon atoms in each alkyl part, nitro, cyano and alkoxycarbonyl with 1 to 4 carbon atoms in the alkoxy part and optionally halogen-substituted phenyl or phenoxy ;

$R^2$ represents straight-chain or branched alkyl with 1 to 6 carbon atoms,

$R^3$ represents straight-chain or branched alkyl with 1 to 4 carbon atoms, chlorine or bromine,

m represents the numbers 1 or 2 and

n represents the numbers 3, 4, 5, 6 and 7,

characterised in that halogenomethyl ketones of the formula II

$$R—CO—CH_2—Hal \qquad (II)$$

in which

R has the abovementioned meaning and

Hal represents chlorine or bromine,

are reacted with azoles of the formula III

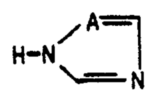

(III)

in which A has the abovementioned meaning, in the presence of a diluent and in the presence of an acid-binding agent.

4. Use of azolylmethyl ketones of the general formula I in Claims 1 and 2 as intermediates for the preparation of hydroxyalkinylazolyl derivatives with fungicidal activity.

**Revendications**

1. Azolylméthyl-cétones de formule générale I :

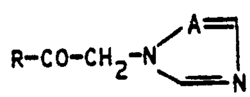

(I)

dans laquelle

A représente un atome d'azote ou le groupe CH,

R représente un groupe adamantyle ou les groupements

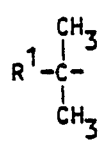

et

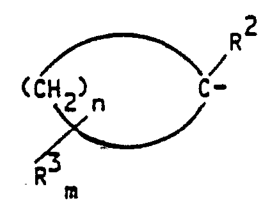

,

où

$R^1$ représente un groupe néopentyle, cyanéthyle ou un groupe phénoxyéthyle éventuellement substitué, de façon identique ou différente, 1 à 3 fois, et l'on peut citer comme substituants du groupe phényle : un atome d'halogène, un groupe alkyle ayant 1 à 4 atomes de carbone, alcoxy et alkylthio ayant chacun 1 à 2 atomes de carbone, halogénoalkyle, halogéno-alcoxy et halogénoalkylthio ayant chacun 1 ou 2 atomes de carbone et 1 à 5 atomes d'halogène identiques ou différents, un groupe cyclohexyle, dialkylamino ayant 1 à 4 atomes de carbone dans chaque partie alkyle, un groupe nitro, cyano, ainsi qu'un groupe alcoxycarbonyle contenant 1 à 4 atomes de carbone dans la partie alcoxy et un groupe phényle ou phénoxy éventuellement substitué par de l'halogène ;

$R^2$ représente un groupe alkyle linéaire ou ramifié ayant 1 à 6 atomes de carbone,

$R^3$ représente un groupe alkyle linéaire ou ramifié ayant 1 à 4 atomes de carbone, ou un atome de chlore ou de brome,

m représente les nombres 1 ou 2, et

n représente les nombres 3, 4, 5, 6 et 7.

2. Composés de formule générale I selon la revendication 1, dans laquelle R représente un groupe adamantyle ou les groupements

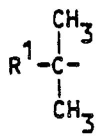 et 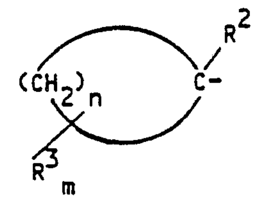 ,

$R^1$ représente un groupe néopentyle, cyanéthyle ou un groupe phénoxyéthyle éventuellement substitué, de façon identique ou différente, 1 à 2 fois, et l'on peut citer comme substituants du groupe phényle : un atome de fluor, de chlore ou de brome, un groupe méthyle, éthyle, isopropyle, tertiobutyle, méthoxy, éthoxy, méthylthio, trifluorométhyle, trifluorométhoxy, trifluorométhylthio, nitro, diméthylamino, méthoxycarbonyle ainsi qu'un groupe phényle ou phénoxy éventuellement substitué par du chlore ;

$R^2$ représente un groupe méthyle ou éthyle ;

$R^3$ représente un groupe méthyle, éthyle, isopropyle, tertiobutyle ou un atome de chlore ;

m représente les nombres 1 ou 2 ; et

A représente un atome d'azote ou le groupe CH, et,

n représente les nombres 3, 4, 5, 6 et 7.

3. Procédé pour préparer des azolylméthyl-cétones de formule générale I

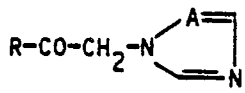 (I)

dans laquelle

A représente un atome d'azote ou le groupe CH,

R représente un groupe adamantyle ou les groupements

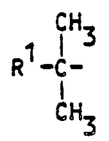 et 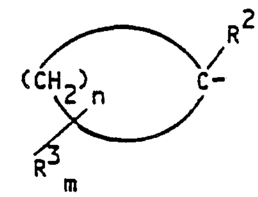 ,

où

$R^1$ représente un groupe néopentyle, cyanéthyle ou un groupe phénoxyéthyle éventuellement substitué, de façon identique ou différente, 1 à 3 fois, et l'on peut citer comme substituants du groupe phényle : un atome d'halogène, un groupe alkyle ayant 1 à 4 atomes de carbone, un groupe alcoxy et alkylthio ayant chacun 1 à 2 atomes de carbone, un groupe halogénoalkyle, halogénoalcoxy et halogénoalkylthio ayant chacun 1 à 2 atomes de carbone et 1 à 5 atomes d'halogène, identiques ou différents, un groupe cyclohexyle, dialkylamino ayant 1 à 4 atomes de carbone dans chaque partie alkyle, un groupe nitro, cyano, ainsi qu'un groupe alcoxycarbonyle ayant 1 à 4 atomes de carbone dans la partie alcoxy et un groupe phényle ou phénoxy éventuellement substitué par de l'halogène ;

$R^2$ représente un groupe alkyle linéaire ou ramifié ayant 1 à 6 atomes de carbone,

$R^3$ représente un groupe alkyle linéaire ou ramifié ayant 1 à 4 atomes de carbone ou un atome de chlore ou de brome,

m représente les nombres 1 ou 2, et

n représente les nombres 3, 4, 5, 6 et 7,

procédé caractérisé en ce qu'on fait réagir des halogénométhyl-cétones de formule II

$$R—CO—CH_2—Hal \qquad (II)$$

dans laquelle

R a le sens indiqué ci-dessus et

Hal représente le chlore ou le brome,

avec des azoles de formule III

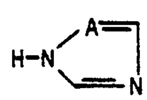 (III)

dans laquelle A a le sens précité, en opérant en présence d'un diluant et en présence d'un agent de fixation des acides.

4. Utilisation des azolylméthyl-cétones de formule générale I selon les revendications 1 et 2 comme produits intermédiaires pour préparer des dérivés hydroxyalcynyl-azolyliques ayant une efficacité fongicide.